# EUROPEAN PATENT APPLICATION

(11) **EP 4 530 349 A1**
(43) Date of publication of application: **02.04.2025**
(21) Application number: 22948717.8
(22) Date of filing: 02.09.2022
(51) Int. Cl.: C12N 11/10, C12N 1/21, C12P 7/18, C12N 15/70

(54) **IMMOBILIZED ENZYME AND USE THEREOF AND METHOD THEREFOR FOR PREPARING ETHYLHEXYLGLYCERIN**

(30) Priority: 27.06.2022 CN 202210737367
(71) Applicant: Nanjing Ascend Megabio Technology Co., Ltd., Nanjing, Jiangsu 210000 (CN)
(72) Inventor: ZHANG, Shasha, Nanjing, Jiangsu 210000 (CN); SHI, Luqiu, Nanjing, Jiangsu 210000 (CN); SU, Guizhen, Nanjing, Jiangsu 210000 (CN)
(74) Representative: Miller, Ronald Anthony
(86) International application number: PCT/CN2022/104609
(87) International publication number: WO 2024/000620

(57) **Abstract**

Disclosed are a fusion protein and an immobilized enzyme including the fusion protein, where the fusion protein includes an epoxide hydrolase active domain and a chitin protein binding domain. The present disclosure achieves efficient immobilization of epoxide hydrolase through the specific affinity between chitin and the fusion protein, and uses ethylhexyl glycidyl ether as a substrate to efficiently produce ethylhexylglycerin with the immobilized enzyme. The immobilization method of the present disclosure offers advantages such as low cost, high enzyme immobilization efficiency, minimal enzyme activity loss, and strong specificity, fundamentally solving issues like poor recyclability and low stability of free enzymes, as well as low repeatability in traditional whole-cell immobilization methods. It resolves issues such as deep color and protein residues in downstream separation and purification.

## Description

### TECHNICAL FIELD

The present disclosure relates to the technical field of biocatalysis, and specifically to an immobilized enzyme and a use thereof and a method therefor for preparing ethylhexylglycerin.

### BACKGROUND

2-ethylhexylglycerin (CasNo: 70445-33-9), also known as isooctyl glycerol ether or octyloxy glycerol, is an amphiphilic molecule with effects such as lubrication, antibacterial, moisturizing, and deodorization. Ethylhexylglycerin is a widely used daily chemical product worldwide, applied in various bathing products, cleaning products, deodorants, eye makeup, foundation, hair care products, and sunscreen products. Ethylhexylglycerin can enhance the moisturizing effect of emollient and moisturizing formulations while providing a smooth skin feel. When incorporated into cream formulations, it increases skin absorption efficiency of the cream and mitigates undesirable sensory effects such as stickiness and whitening. The antibacterial activity of ethylhexylglycerin can inhibit the growth and reproduction of bacteria that cause odor on the skin surface, providing a deodorizing effect. In addition, the combination of ethylhexylglycerin with traditional preservatives, such as phenoxyethanol, has a significant preservative synergistic function. This feature can effectively reduce the amount of traditional preservatives used, significantly lower the toxicity of the preservative system, making cosmetics safer and more reassuring for consumers. Ethylhexylglycerin has become an important variety of green preservatives, with broad applications and large consumption.

Although ethylhexylglycerin is a green preservative, Chinese Patent No. CN108191614A discloses a method for preparing ethylhexylglycerin, which includes the following steps: (1) Using boron trifluoride etherate solution as a catalyst, under stirring conditions, react isooctanol and glycidyl esters at room temperature to generate an intermediate; (2) Adding an acid as a catalyst to the intermediate obtained in step (1), then adding water or alcohol, reacting under atmospheric or reduced pressure to hydrolyze or alcoholize the intermediate obtained in step (1); (3) Washing and separating the product obtained in step (2), then distilling to obtain ethylhexylglycerin. However, this method results in side reactions during synthesis, producing many impurities. The presence of impurities in the chemical synthesis process can lead to deep color, odor, and sometimes toxic reactions in the produced ethylhexylglycerin. To remove these impurities, steps such as decolorization and impurity removal need to be added in the post-processing process, further increasing production costs.

Biological enzyme methods are characterized by high catalytic efficiency and good specificity. The immobilized enzyme form allows for repeated recycling of enzymes, facilitates separation from substrates and products after the reaction, enhances stability against heat, pH, etc., with no enzyme residue in the product solution, simplifying the purification process. Immobilized enzymes are more suitable for industrial applications. Common methods of enzyme immobilization generally involve obtaining purified free enzymes, then immobilizing them by adsorption, embedding, covalent bonding, etc., which are cumbersome processes with low enzyme activity recovery rates. Thus, a highly specific, efficient, low-cost enzyme immobilization method is of significant importance.

### SUMMARY

The objective of the present disclosure is to provide a method for preparing ethylhexylglycerin using an immobilized enzyme. The present disclosure identifies a chitin-binding domain with chitin-binding activity, leveraging the specific and efficient binding properties of the polypeptide to chitin, thereby achieving simultaneous enzyme purification and immobilization. The use of this immobilized enzyme in the production of ethylhexylglycerin achieves significant cost savings.

In order to achieve the above objective, the present disclosure provides the following technical solutions.

In the first aspect, the present disclosure provides a fusion protein including an epoxide hydrolase active domain and a chitin protein binding domain.

Further, the epoxide hydrolase active domain is an expression product of SEQ ID NO: 1 or an amino acid sequence obtained by substitution, deletion, or addition of one or more amino acids of the expression product, or an amino acid sequence with at least 80% sequence identity to the expression product.

Further, the chitin protein binding domain is an expression product of SEQ ID NO: 2 or an amino acid sequence obtained by substitution, deletion, or addition of one or more amino acids of the expression product, or an amino acid sequence with at least 80% sequence identity to the expression product.

Preferably, the fusion protein further includes a linker peptide, which is an expression product of SEQ ID NO: 3 or an amino acid sequence obtained by substitution, deletion, or addition of one or more amino acids of the expression product, or an amino acid sequence with at least 80% sequence identity to the expression product.

Specifically, the fusion protein is an expression product of SEQ ID NO: 4 or an amino acid sequence obtained by substitution, deletion, or addition of one or more amino acids of the expression product, or an amino acid sequence with at least 80% sequence identity to the expression product.

In the second aspect, the present disclosure provides an immobilized enzyme, which includes a chitin immobilized carrier and the fusion protein as described above.

Preferably, the chitin immobilized carrier is selected from any one or more of chitin powder, chitin particles, chitin resin, chitin magnetic beads, chitin polymer, or chitin gel.

In the third aspect, the present disclosure further provides a vector including the DNA sequences shown in SEQ ID NO: 1 and SEQ ID NO: 2. More preferably, the vector includes the DNA sequence shown in SEQ ID NO: 4.

In the fourth aspect, the present disclosure provides a genetically engineered cell, including the DNA sequences shown in SEQ ID NO: 1 and SEQ ID NO: 2 in a free or integrated state. More preferably, the genetically engineered cell includes the DNA sequence shown in SEQ ID NO: 4 in a free or integrated state.

In the fifth aspect, the present disclosure provides a method for preparing an immobilized enzyme, including:
1) constructing a vector, where the vector includes any one of the vectors described in the third aspect;
2) transforming the host cell with the constructed vector from step 1), to obtain recombinant cells;
3) culturing the recombinant cells from step 2) to obtain a fusion protein including an epoxide hydrolase active domain and a chitin protein binding domain;
4) mixing the fusion protein obtained from step 3) with a chitin immobilized carrier to obtain an immobilized enzyme,

where the epoxide hydrolase active domain is an expression product of SEQ ID NO: 1 or an amino acid sequence obtained by substitution, deletion, or addition of one or more amino acids of the expression product, or an amino acid sequence with at least 80% sequence identity to the expression product; the chitin protein binding domain is an expression product of SEQ ID NO: 2 or an amino acid sequence obtained by substitution, deletion, or addition of one or more amino acids of the expression product, or an amino acid sequence with at least 80% sequence identity to the expression product.

Preferably, the host cell is a bacterial or fungal cell.

In the sixth aspect, the present disclosure provides the use of the fusion protein or immobilized enzyme as described above or the immobilized enzyme prepared according to the above method, for the preparation of ethylhexylglycerin with ethylhexyl glycidyl ether as a substrate.

In the seventh aspect, the present disclosure provides a method for preparing ethylhexylglycerin, including using ethylhexyl glycidyl ether as a substrate and contacting it with the fusion protein or immobilized enzyme as described above or the immobilized enzyme prepared according to the above method to produce ethylhexylglycerin.

Compared with the prior art, the advantages of the present disclosure are as follows. The present disclosure discloses a fusion protein and an immobilized enzyme including the fusion protein, where the fusion protein includes an epoxide hydrolase active domain and a chitin protein binding domain. The present disclosure achieves efficient immobilization of epoxide hydrolase through the specific affinity between chitin and the fusion protein, and uses ethylhexyl glycidyl ether as a substrate to efficiently produce ethylhexylglycerin with the immobilized enzyme. The immobilization method of the present disclosure offers advantages such as low cost, high enzyme immobilization efficiency, minimal enzyme activity loss, and strong specificity, fundamentally solving issues like poor recyclability and low stability of free enzymes, as well as low repeatability in traditional whole-cell immobilization methods. It resolves issues such as deep color and protein residues in downstream separation and purification.

### BRIEF DESCRIPTION OF THE DRAWINGS

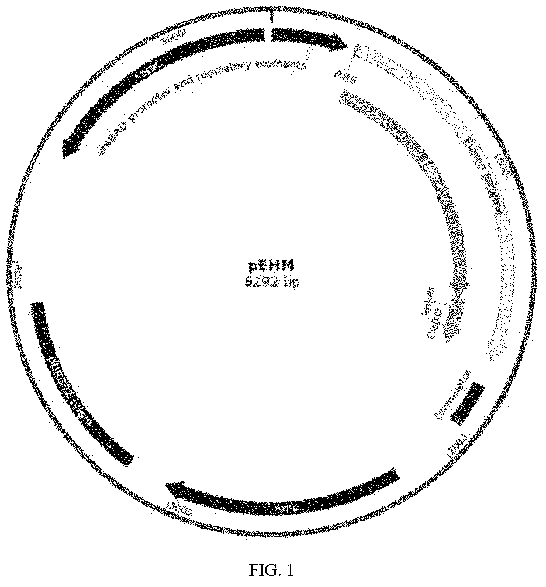

FIG. 1 shows a physical map of the recombinant expression vector constructed in the present disclosure.

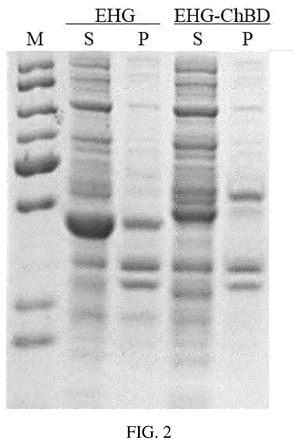

FIG. 2 shows the expression of the fusion protein, where M is the marker, EHG is the epoxide hydrolase protein, and EHG-ChBD is the fusion protein.

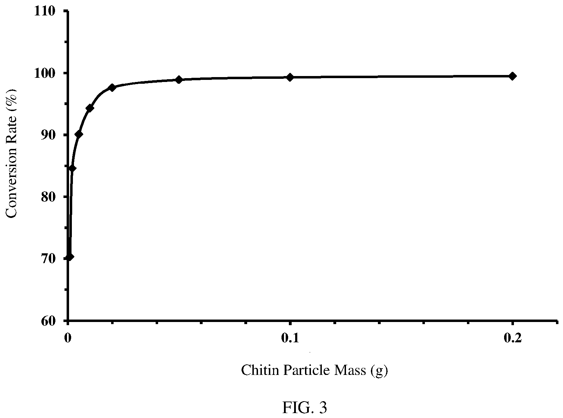

FIG. 3 shows the loading amount of the recombinant epoxide hydrolase on chitin particles.
FIG. 4 shows the number of reuse batches and conversion rate of the immobilized enzyme.
FIG. 5 shows the gas chromatographic analysis of ethylhexylglycerin prepared by catalysis of 50% ethylhexyl glycidyl ether using the immobilized enzyme.

### Sequence Listing Description:

SEQ ID NO: 1: DNA sequence of the epoxide hydrolase (NaEH) gene from Novosphingobium aromaticivorans;
SEQ ID NO: 2: DNA sequence of the chitin-binding protein (L1ChBD) gene from Lactococcus lactis;
SEQ ID NO: 3: DNA sequence of a synthetically produced linker peptide;
SEQ ID NO: 4: DNA sequence of the fusion protein of the present disclosure;
SEQ ID NO: 5: NaEH forward primer;
SEQ ID NO: 6: NaEH reverse primer;
SEQ ID NO: 7: L1ChBD forward primer;
SEQ ID NO: 8: L1ChBD reverse primer; pBAD
SEQ ID NO: 9: Verification for forward primer;
SEQ ID NO: 10: Verification for reverse primer.

### DETAILED DESCRIPTION OF THE EMBODIMENTS

The technical solutions in the specific embodiments of the present disclosure are clearly and completely described in conjunction with the accompanying drawings in the embodiments of the present disclosure. It is understood that the described embodiments are only a part of the embodiments of the present disclosure, instead of all embodiments. All other embodiments, which can be derived by those skilled in the art from the embodiments given herein without making any creative effort, shall fall within the protection scope of the present disclosure.

In the description of the present disclosure, it is necessary to note that terms such as "upper," "lower," "inner," "outer," "front end," "rear end," "both ends," "one end," "the other end," etc., indicate orientation or positional relationships based on the orientation or positional relationships shown in the drawings, only for convenience in describing the present disclosure and simplifying the description, and are not to be construed as indicating or implying that the referenced devices or elements must have specific orientations, be constructed in a specific orientation, or operate in a specific orientation, and thus should not be construed as limitations of the present disclosure. Additionally, terms such as "first" and "second" are used for descriptive purposes only and should not be understood as indicating or implying relative importance.

The present disclosure discloses a fusion protein, an immobilized enzyme containing the fusion protein, and a method for preparing ethylhexylglycerin by using this immobilized enzyme. The present disclosure identifies a chitin-binding domain with chitin-binding activity, leveraging the specific and efficient binding properties of the polypeptide to chitin, thereby achieving simultaneous enzyme purification and immobilization. The use of this immobilized enzyme in the production of ethylhexylglycerin achieves significant cost savings.

The method for preparing ethylhexylglycerin using the immobilized enzyme of the present disclosure includes:
(1) Mixing the supernatant containing the fusion protein with chitin to achieve enzyme immobilization and purification;
(2) The substrate for catalysis by the immobilized enzyme is ethylhexyl glycidyl ether, with a substrate concentration range of 5-50% (W/V);
(3) Resuspending the immobilized enzyme with a phosphate buffer of 20mM, pH range 6.0-9.0, and adding ethylhexyl glycidyl ether;
(4) The amount of immobilized enzyme per mL of reaction system is 0.01-0.2g;
(5) The conversion reaction temperature is 30-50° C; the reaction time is 4-8 hours to produce ethylhexylglycerin.

By adopting the above technical solution, 500g/L of ethylhexyl glycidyl ether can be fully converted to ethylhexylglycerin within 8 hours, without any by-products, demonstrating good industrial application potential and achieving the purpose of reducing impurities and saving costs.

Another objective of the present disclosure is to provide a method for preparing the immobilized enzyme described above for preparing ethylhexylglycerin, which includes:
(1) Activating the genetically engineered strain of high-yield epoxide hydrolase fusion protein by overnight culture in seed medium at 37° C, inoculating it at a 10% ratio into fermentation medium, culturing at 37° C, 200rpm until mid-log phase, adding the inducer arabinose to a final concentration of 1g/L, and inducing at 25-30° C, 200 rpm for about 6-8 hours;
(2) Centrifuging to collect cells, resuspending cells in a concentration of 0.01-0.1g wet cells/mL with 10mM sodium phosphate buffer at pH 7.0, and sonically disrupting them for 10 minutes in an ice bath;
(3) Centrifuging the disrupted cell solution at 12000rpm for 10 minutes, and taking the supernatant as the crude enzyme solution of the recombinant epoxide hydrolase for immobilization;
(4) Mixing the obtained supernatant with chitin for incubation to obtain immobilized epoxide hydrolase.

By adopting the above technical solution, the immobilized enzyme exhibits high activity and tolerance to high substrate concentration, resulting in high conversion reaction efficiency and 100% substrate conversion rate.

The present disclosure further specifies that the seed medium is a conventional LB medium with the following formulation: Peptone 10g/L, yeast extract 5g/L, NaCl 10g/L; the plate medium is LB medium with 15g/L agar added and sterilized at 121° C for 20 minutes.

The present disclosure further specifies that the fermentation medium is a conventional TB medium with the following formulation: Peptone 12g/L, yeast extract 24g/L, glycerol 4mL/L, diluted to 900mL, and 100mL of 0.17M potassium dihydrogen phosphate-dipotassium hydrogen phosphate solution prepared, sterilized at 115° C for 20 minutes, mixed after sterilization.

Another objective of the present disclosure is to provide a method for producing a genetically engineered strain for high-yield fusion epoxide hydrolase, as described in the method for preparing the immobilized enzyme above. This method involves obtaining the epoxide hydrolase (NaEH) gene from Novosphingobium aromaticivorans via PCR, with the gene product sequence designated as ABD26703.1 and the nucleotide sequence as shown in SEQ ID NO: 1. The chitin-binding protein (LlChBD) gene from Lactococcus lactis is also obtained via PCR, with the nucleotide sequence as shown in SEQ ID NO: 2. A linker peptide is introduced through primers, with the nucleotide sequence as shown in SEQ ID NO: 3. The NaEH gene, linker coding sequence, and L1ChBD gene are inserted between the NcoI and EcoRI sites of the plasmid pYB1s to obtain the recombinant expression vector, designated as pEHM. The recombinant expression vector was then transformed into host bacteria Escherichia coli K-12 BW 25113, thus obtaining the genetically engineered strain for high-yield epoxide hydrolase fusion protein.

By adopting the above technical solution, the target protein expressed by the genetically engineered strain of high-yield epoxide hydrolase fusion protein can reach 20-40% of total protein, achieving efficient heterologous expression of the target protein.

The present disclosure further specifies that the donor bacteria are from the genera Escherichia, Corynebacterium, Bacillus, Pseudomonas, Saccharomyces, and Fusarium, containing the NaEH gene.

Another objective of the present disclosure is to provide an application of the genetically engineered strain for the high-yield epoxide hydrolase fusion protein as described above, in the method for preparing ethylhexylglycerin using an immobilized enzyme.

In summary, the present disclosure has the following beneficial effects: The immobilized enzyme catalyzes 100% substrate conversion rate. Compared to the current industrial chemical method of preparing ethylhexylglycerin, it has a simple process, is environmentally friendly, generates no by-products, can significantly reduce the use of acids, bases, and organic solvents, has high production efficiency, no enzyme residue in the product solution, and a simplified purification process.

In order to make the technical means, the creation features, the achievement purposes and the effects of the present disclosure easy to understand, the technical proposals in the embodiments of the present disclosure will be clearly and completely described below with reference to the embodiments and drawings of the present disclosure, and it is obvious that the described embodiments are only a part but not all of the embodiments of the present disclosure. All other embodiments, which can be derived by those skilled in the art from the embodiments given herein without making any creative effort, shall fall within the protection scope of the present disclosure.

In the examples of the present disclosure, the experimental methods used are all conventional methods unless otherwise specified.

The materials and reagents used in the examples of the present disclosure are commercially available unless otherwise specified.

As used in this disclosure, the terms "polypeptide," "peptide," and "protein" are used interchangeably to refer to amino acid polymers of any length.

As used in this disclosure, the term "chitin-binding domain" refers to a polypeptide capable of specifically binding to chitin or chitin, generally of small molecular weight (30-100 amino acids), and also has good stability, capable of binding to chitin or chitin in harsh environments, thus having good application in the purification and immobilization of target proteins (enzymes).

The vector described in the following examples is the pBAD/HisB vector.

### Example 1: Construction of the expression vector for epoxide hydrolase with a chitin-binding domain

Using the genomes of Novosphingobium aromaticivorans and Lactococcus lactis as templates, the epoxide hydrolase gene NaEH and the chitin-binding protein gene LlChBD were obtained by PCR amplification using primer pairs EH-F and EH-R, Ll-F and L1-R. The pBAD/HisB vector was double-digested with NcoI and EcoRI, and the large fragment of the vector, approximately 3960bp, was recovered. The recovered NaEH and LlChBD gene fragments and the large vector fragment were ligated using the Gibson method (Gibson DG, Young L, Chuang RY, Venter JC, Hutchison CA, 3rd, Smith HO: Enzymatic assembly of DNA molecules up to several hundred kilobases. Nat Methods 2009, 6:343-345.), and the ligation product was transformed into Fast-T1 competent cells (Nanjing Novozymes Biotechnology Co., Ltd., catalog number C505) and plated on LB agar containing streptomycin. After overnight incubation at 37° C, single colonies were picked for plasmid extraction, and a pair of primers (pBAD-F and pBAD-R) was designed for PCR verification, with correct clones sent for sequencing. The recombinant vector obtained by replacing the fragment between the NcoI and EcoRI sites of the pBAD/HisB vector with the epoxide hydrolase gene NaEH shown in SEQ ID NO: 1, the chitin-binding domain gene L1ChBD shown in SEQ ID NO: 2, and the linker peptide gene shown in SEQ ID NO: 3 was named pEHM. The primer sequences are as follows:
EHG-F SEQ ID NO: 5 :
   5'-GCTAACAGGAGGAATTAACCATGGATGTTGCGCCTTTCGTTG-3'
EHG-R SEQ ID NO: 6 :
   5'-CCACCACCTCCTGATCCACCACCTCCTGATCCACCACCTCCGCACATCAGGGAAAAC

GCG-3', the underlined sequence is the linker peptide sequence
LL-F SEQ ID NO: 7 :
5'-GGTGGATCAGGAGGTGGTGGATCAGAAGCTGCAGCTAAGACTACTTATACCGTCAA

ATCT-3', the underlined sequence is the linker peptide sequence
LL-R SEQ ID NO: 8 :
5'-GCTGCAGACCGAGCTCACCGAATTCTTATGTCAGTACAAGTTTTTGACCAATG-3'

| | | |
|---|---|---|
| pBAD-F | SEQ ID NO: 9: | 5'-CGGCGTCACACTTTGCTATG-3' |
| pBAD-R | SEQ ID NO: 10: | 5'-CGTTTCACTTCTGAGTTCGGC-3' |

In the gene expression cassette for the epoxide hydrolase fusion protein, the promoter initiating transcription of the epoxide hydrolase fusion protein gene is the pBAD promoter.

### Example 2 Construction of engineered strain BW/pEHM for high-yield epoxide hydrolase fusion protein

The expression vector pEHM constructed in Example 1 was chemically transformed into Escherichia coli K-12 BW 25113, and positive clones were selected on LB plates containing streptomycin (streptomycin concentration 50ug/mL), yielding an engineered strain named BW/pEHM.

Example 3 Preparation of recombinant epoxide hydrolase with chitin-binding domain
(1) The engineered strain BW/pEHM obtained in Example 2 was inoculated in 5mL LB (Str50 µg/mL) seed medium, activated overnight at 37° C, 200rpm.
(2) The overnight-activated seed culture was inoculated at a 10% ratio into 500mL TB fermentation medium (Str50 µg/mL), cultured at 37° C, 200rpm until mid-log phase, arabinose (inducer) was added to a final concentration of 1g/L, and induced at 25-30° C, 200rpm for about 6-8 hours;
(3) The cells were collected by centrifugation at 8000rpm for 10 minutes, resuspended in 100mL of 10mM sodium phosphate buffer at pH 7.0, and sonically disrupted for 10 minutes in an ice bath. The disrupted cell solution was centrifuged at 12000rpm/min for 10 minutes, and the supernatant was taken as the crude enzyme solution of the recombinant epoxide hydrolase fusion protein for immobilization or freeze-drying for standby application.

The composition of the LB medium is: Peptone 10g/L, yeast extract 5g/L, NaCl 10g/L; the plate medium is LB medium with 15g/L agar added and sterilized at 121° C for 20 minutes.

The composition of the TB medium is: Peptone 12g/L, yeast extract 24g/L, glycerol 4mL/L, diluted to 900mL, and 100mL of 0.17M potassium dihydrogen phosphate-dipotassium hydrogen phosphate solution prepared, sterilized at 115° C for 20 minutes, mixed after sterilization.

### Example 4 Preparation of immobilized epoxide hydrolase

Mixing the above recombinant epoxide hydrolase with chitin powder: Taking 2mL of the crude enzyme solution of recombinant epoxide hydrolase fusion protein obtained in Example 3, adding chitin particles of different weights, incubating at 250rpm within a temperature range of room temperature to 40° C for 1 hour to achieve one-step immobilization and purification of the recombinant epoxide hydrolase on chitin particles. Low-temperature high-speed centrifugation (10000rpm, 4° C, 10min) was used to separate the immobilized enzyme from the liquid, with the excess supernatant being discarded. The immobilized enzyme was resuspended with 20mM sodium phosphate buffer at pH 7.0, centrifuged (10000rpm, 4° C, 10 min) to remove the supernatant, and the operation was repeated multiple times until no protein remained in the supernatant (detected by Coomassie brilliant blue solution) to remove non-specifically binding proteins. The remaining chitin particles after the removal of supernatant were the immobilized epoxide hydrolase. The immobilized epoxide hydrolase was freeze-dried to obtain dry immobilized epoxide hydrolase, which can be stored for long-term use.

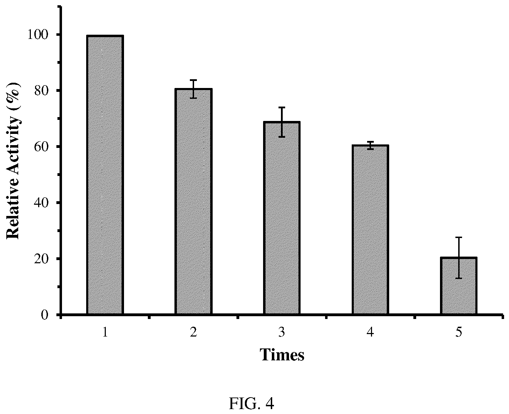

Testing the reuse batches of immobilized epoxide hydrolase: The first batch of immobilized epoxide hydrolase was used for reaction, and the conversion rate of the first batch was recorded. After activity measurement, the immobilized enzyme was rinsed with buffer to remove any residual reaction solution. The reaction was repeated for five batches, and the reuse frequency and conversion rate were calculated. The test results showed that, with the increase of reaction batches, the conversion rate gradually decreased. The conversion rate could still remain above 80% after two consecutive batches were used. The test results are shown in FIG. 4.

### Example 5 Immobilized epoxide hydrolase used for preparing ethylhexylglycerin

The 0.1g of immobilized epoxide hydrolase was taken and suspended in 1mL of 20mM sodium phosphate buffer at pH 7.0, and 1mL of ethylhexyl glycidyl ether substrate was added. The conversion reaction temperature was 37° C, and the conversion reaction was conducted at 200rpm for 4 hours, producing ethylhexylglycerin. The using amount of immobilized enzyme per mL of reaction system was 0.01-0.2g.

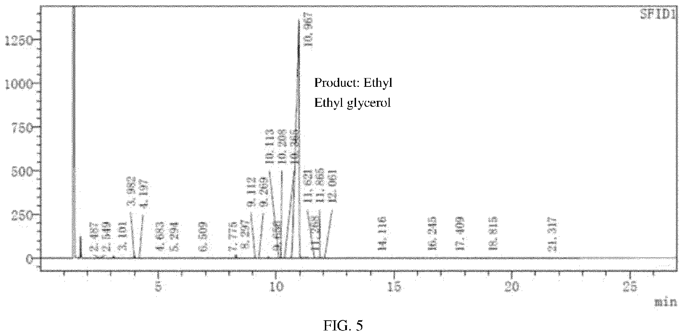

The conversion rate and purity of the ethylhexylglycerin product were detected using gas chromatography analysis, and the results are shown in FIG. 5.

The chromatographic column used for gas chromatography analysis was Agilent HP-5 (0.2mm × 30m), with an initial temperature of 100° C, a temperature ramp rate of 25° C/min, and an injection volume of 2 µL.

Any aspects not detailed in the present disclosure are deemed to be common knowledge among those skilled in the art.

Finally, it should be noted that the specific embodiments described above are intended to illustrate the technical solutions of the present disclosure rather than to limit them. Although the present disclosure has been described in detail with reference to the embodiments, those skilled in the art should understand that modifications and equivalent replacements can be made to the technical solutions of the present disclosure without departing from the spirit and scope of the technical solutions, all of which should be covered within the scope of the claims of the present disclosure.

## Claims

1. A fusion protein, **characterized by** comprising an epoxide hydrolase active domain and a chitin protein binding domain.

2. The fusion protein according to claim 1, **characterized in that** the epoxide hydrolase active domain is an expression product of SEQ ID NO: 1 or an amino acid sequence obtained by substitution, deletion, or addition of one or more amino acids of the expression product, or an amino acid sequence with at least 80% sequence identity to the expression product.

3. The fusion protein according to claim 1, **characterized in that** the chitin protein binding domain is an expression product of SEQ ID NO: 2 or an amino acid sequence obtained by substitution, deletion, or addition of one or more amino acids of the expression product, or an amino acid sequence with at least 80% sequence identity to the expression product.

4. The fusion protein according to claim 1, **characterized by** further comprising a linker peptide, wherein the linker peptide is an expression product of SEQ ID NO: 3 or an amino acid sequence obtained by substitution, deletion, or addition of one or more amino acids of the expression product, or an amino acid sequence with at least 80% sequence identity to the expression product.

5. The fusion protein according to claim 1, **characterized in that** the fusion protein is an expression product of SEQ ID NO: 4 or an amino acid sequence obtained by substitution, deletion, or addition of one or more amino acids of the expression product, or an amino acid sequence with at least 80% sequence identity to the expression product.

6. An immobilized enzyme, **characterized by** comprising a chitin immobilized carrier and the fusion protein according to any one of claims 1 to 5.

7. The immobilized enzyme according to claim 6, **characterized in that** the chitin immobilized carrier is one or more selected from the group consisting of a chitin powder, a chitin particle, a chitin resin, a chitin magnetic bead, a chitin polymer, and a chitin gel.

8. A vector, **characterized by** comprising a DNA sequence set forth in SEQ ID NO: 1 and a DNA sequence set forth in SEQ ID NO: 2.

9. The vector according to claim 8, **characterized in that** the vector comprises a DNA sequence set forth in SEQ ID NO: 4.

10. A genetically engineered cell, **characterized by** comprising a DNA sequence set forth in SEQ ID NO: 1 and a DNA sequence set forth in SEQ ID NO: 2 each in a free state or in an integrated state.

11. The genetically engineered cell according to claim 10, **characterized in that** the genetically engineered cell comprises a DNA sequence set forth in SEQ ID NO: 4 in a free state or in an integrated state.

12. A method for preparing an immobilized enzyme, **characterized by** comprising:
step 1: constructing a vector, wherein the vector is the vector according to claim 8 or 9;
step 2: transforming a host cell with the vector constructed in the step 1 to obtain a recombinant cell;
step 3: culturing the recombinant cell from the step 2 to obtain a fusion protein, wherein the fusion protein comprises an epoxide hydrolase active domain and a chitin protein binding domain; and
step 4: mixing the fusion protein obtained in the step 3 with a chitin immobilized carrier to obtain the immobilized enzyme,
wherein the epoxide hydrolase active domain is an expression product of SEQ ID NO: 1 or an amino acid sequence obtained by substitution, deletion, or addition of one or more amino acids of the expression product, or an amino acid sequence with at least 80% sequence identity to the expression product; the chitin protein binding domain is an expression product of SEQ ID NO: 2 or an amino acid sequence obtained by substitution, deletion, or addition of one or more amino acids of the expression product, or an amino acid sequence with at least 80% sequence identity to the expression product.

13. The method for preparing the immobilized enzyme according to claim 12, **characterized in that** the host cell is a bacterial cell or a fungal cell.

14. A use of the fusion protein according to any one of claims 1 to 5, or the immobilized enzyme according to claim 6 or 7, or an immobilized enzyme prepared by the method according to claim 12 or 13 in the preparation of ethylhexylglycerin with ethylhexyl glycidyl ether as a substrate.

15. A method for preparing ethylhexylglycerin, **characterized by** comprising: allowing ethylhexyl glycidyl ether as a substrate to come into contact with the fusion protein according to any one of claims 1 to 5 or the immobilized enzyme according to claim 6 or 7 or an immobilized enzyme prepared by the method according to claim 12 or 13 to produce the ethylhexylglycerin.
